(19) Europäisches Patentamt
European Patent Office
Office européen des brevets

(11) Publication number: 0 331 635 A2

(12) **EUROPEAN PATENT APPLICATION**

(21) Application number: 89810133.2

(22) Date of filing: 21.02.89

(51) Int. Cl.4: A 61 K 45/02
C 12 P 21/00, C 07 K 15/26
// A61K9/50

(30) Priority: 29.02.88 US 162241

(43) Date of publication of application:
06.09.89 Bulletin 89/36

(84) Designated Contracting States:
AT BE CH DE FR GB IT LI LU NL SE

(71) Applicant: THE BOARD OF REGENTS UNIVERSITY OF TEXAS SYSTEM
Cancer Center 1515 Holcombe Boulevard
Houston Texas 77030 (US)

(72) Inventor: Fidler, Isaiah, Prof. Dr.
2307 Forest Garden Drive
Kingwood Texas 77345 (US)

Johnson, John , Dr.
3718 Merrick
Houston Texas 77025 (US)

Nayar, Rajiv, Dr.
110 West 4th Street, NBR 401
North Vancouver British Columbia V7M3H3 (CA)

Killion, Jerald Jay, Prof. Dr.
14407 Harvest Ridge Road
Houston Texas 77062 (US)

(74) Representative: Meyer, Hans Rudolf et al
Patentabteilung der CIBA-GEIGY AG Postfach
CH-4002 Basel (CH)

# (54) Method for treating bladder cancer.

(57) The invention concerns a method for the treatment of bladder cancer by applying alpha-interferon (IFN-α) hybrids, or especially liposomes containing such hybrids.

Figure 1

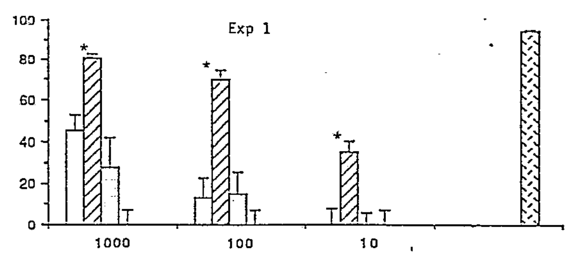


EP 0 331 635 A2

## Description

# Method for Treating Bladder Cancer

Field of the invention

The invention concerns a method for the treatment of bladder cancer by applying alpha-interferon (IFN-α) hybrids, or especially liposomes containing such hybrids.

Background of the invention

Human leukocyte-derived interferons (alpha interferon, IFN-α) consist of at least 14 nonallelic subtypes (1-3). Although interferons were first considered to be mainly potent antiviral agents (4), some have been shown to mediate direct antiproliferative activity against various tumor cell lines in vitro (5-10) and in vivo (48-53). In addition, alpha interferons have been shown to augment host immunity (11), natural killer cell activity (12-16), and monocyte function (17, 18). Interferons have also been shown capable of inducing the expression of major histocompatibility antigens on tumor cells (19) and to induce cell differentiation (20). Specific receptors for alpha, beta, and gamma interferons have been found on mouse (21-23), bovine (24, 25), and human cells (25-30). The receptors for gamma interferon are distinct from those for alpha or beta (31-33). IFN-α is internalized into target cells by receptor-mediated endocytosis (34, 54-56) where it stimulates several intracellular enzymes such as (2′-5′) oligoadenylate synthetase (28, 35), protein kinase (36), and 2′phosphodiesterase (37). Whether the induction of these enzymes contributes to the antiproliferative or antiviral activity of IFN-α is still unclear (7, 9, 38). Different subclasses of IFN-α molecules can bind with different affinities (57). Antiviral activity of some interferon hybrids seems to be directly correlated with binding affinity to target cells (57).

According to EP 205404 genetically engineered IFN-α B/D hybrids exhibit a broad spectrum of antitumor cytostatic activity against several human tumor cell lines (10) such as cancers of the breast, colon and lung, ovarian and melanoma. Specifically, hybrids with the B domain at the N terminus exhibit significantly higher antiproliferative activities than hybrids with the D domain at the N terminal end space. Moreover, the activity of the IFN-α hybrids is due to a direct effect on tumor cells.

An activity against bladder cancer was not yet reported.

The degree of antiproliferative activity of the IFN-α B/D hybrids against the cancer cells depents on the type of the hybrid and the type of cancer cell, and it cannot be foreseen without testing whether particular types of cancer cells will be affected or not.

Surprisingly it was now found out that human bladder cancer cells of various origin respond very well to treatment with certain types of IFN-α B/D hybrids.

Object of the invention

It is an object of the invention to provide a method for the treatment of bladder cancer by administration of IFN-α B/D hybrids.

Detailed description of the invention

The invention in particular concerns a therapeutical method for the treatment of bladder cancer, characterised in that an IFN-α BBDD or BBDB hybrid in a cytostatically effective amount is administered to a patient suffering from bladder cancer.

By IFN-α BBDD is intended the interferon-α hybrid B/D as described in EP 32/34 (equivalent to USP 4,414,150) or the hybrid $B_1B_2D_3D_4$ as described in EP 205404. The IFN-α BBDB is in particular the interferon-α hybrid $B_1B_2D_3B_4$ described in EP 205404.

The bladder cancer to be treated can be of superficial and/or invasive and metastatic nature. For treatment of superficial bladder cancer a present hybrid IFN is administered preferably directly into the bladder (intrabladder), e.g. by a catheder.

Other administration forms are parenteral, e.g. intravenous or intramuscular, administrations which are preferably used in the treatment of invasive and metastatic bladder cancer.

The present hybrids are administered in form of pharmaceutical preparations suitable for intrabladder or parenteral administration.

Such preparations are especially fluids that are administered in various manners, such as intravenously, intramuscularly, intraperitoneally, intranasally, intradermally, subcutaneously or in particular intrabladderly. Such fluids are preferably isotonic aqueous solutions or suspensions which can be prepared before use, for example from lyophilised preparations which contain the active ingredient alone or together with a pharmaceutically acceptable carrier. The pharmaceutical preparations may be sterilised and/or contain adjuncts, for example preservatives, stabilisers, wetting agents and/or emulsifiers, solubilisers, salts for regulating the osmotic pressure and/or buffers.

Of particular importance are pharmaceutical liposome preparations containing the present hybrids. The liposome preparations are particularly suited for bladder cancer treatment as they surprisingly attach preferably to bladder cancer cells as compared to non-cancerous cells.

The present pharmaceutical preparations, which may, if desired, contain further pharmacologically valuable

substances, are produced in a manner known per se, for example by means of conventional dissolving or lyophilising processes, and contain from approximately 0.1% to 100%, especially from approximately 1% to approximately 50%, and in the case of lyophilisates up to 100%, of active ingredient.

Object of the present invention are pharmaceutical compositions whose components, when applied in the form of liposomes, are being enriched or concentrated at bladder cancer cells.

The present invention relates also to pharmaceutical liposome compositions consisting of

a) a IFN-α BBDD or IFN-α BBDB hybrid and

b) a phospholipid of the formula

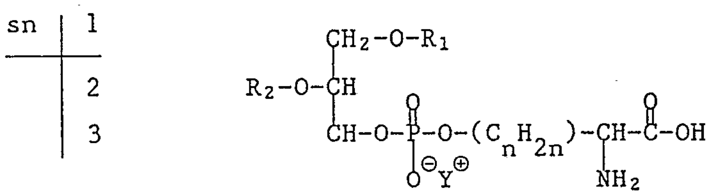

(I),

wherein n is one, two or three, $R_1$ and $R_2$ independently of each other represent alkyl, alkenyl or acyl each having 10-20 carbon atoms, and $Y^{\oplus}$ is the cation of a pharmaceutically acceptable base,

c) a phospholipid of the formula

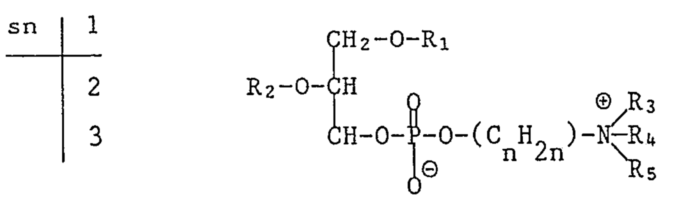

(II),

wherein n is two, three or four, $R_1$ and $R_2$ are defined as above and $R_3$, $R_4$ and $R_5$ represent hydrogen or $C_1$-$C_4$-alkyl, and, optionally, a pharmaceutically acceptable carrier solution buffered to pH 7.0-7.8 and, optionally, pharmaceutically acceptable carriers in solid form.

The nomenclature of the phospholipids of the formulae I and II is in agreement with the recommendations of the IUPAC and IUB Commission on Biochemical Nomenclature (CBN) according to the Eur. J. of Biochem. 79, 11-21 (1977) "Nomenclature of Lipids" (sn-nomenclature stereospecific numbering).

$R_3$, $R_4$ and $R_5$ as $C_1$-$C_4$-alkyl are, for example, ethyl, n-propyl or isopropyl or, preferably, methyl.

In a phospholipid of the formula I (component b)) the group -($C_nH_{2n}$)- is straight chained or branched alkylene, for example 1,1-or 1,2-ethylene, 1,1-, 1,2- or 1,3-propylene or, preferably, methylene (n=1).

Alkyl $R_1$ and $R_2$ is preferably straight-chained with an even number from 10 to 20 carbon atoms, for example n-decyl, n-dodecyl (lauryl), n-tetradecyl (myristyl), n-hexadecyl (cetyl), n-octadecyl (stearyl) or n-icosyl (arachinyl).

Alkenyl $R_1$ and $R_2$ is preferably straight-chained with an even number from 12 to 20 carbon atoms and a double bond, for example 9-cis-dodecenyl (lauroleyl), 9-cis-tetradecenyl (myristoleyl), 9-cis-hexadecenyl (palmitoleinyl), 6-cis-octadecenyl (petroselinyl), 6-trans-octadecenyl (petroselaidinyl), 9-cis-octadecenyl (oleyl), 9-trans-octadecenyl (elaidinyl) or 9-cis-icosenyl (gadoleinyl).

Acyl $R_1$ and $R_2$ is preferably straight-chained with an even number of 10-20 carbon atoms, for example $C_{10}$-$C_{20}$-alkanoyl or $C_{10}$-$C_{20}$-alkenoyl.

Alkanoyl $R_1$ and $R_2$ is preferably n-decanoyl, n-dodecanoyl (lauroyl), n-tetradecanoyl (myristoyl), n-hexadecanoyl (palmitoyl), n-octadecanoyl (stearoyl) and n-icosanoyl (arachinoyl).

Alkenoyl $R_1$ and $R_2$ is preferably 9-cis-dodecenoyl (lauroleoyl), 9-cis-tetradecenoyl (myristoleoyl), 9-cis-hexadecenoyl (palmitoleoyl), 6-cis-octadecenoyl (petroseleoyl), 6-trans-octadecenoyl (petroselaidoyl), 9-cis-octadecenoyl (oleoyl), 9-trans-octadecenoyl (elaidoyl), 11-cis-octadecenoyl (vaccenoyl) and 9-cis-icosenoyl (gadoleoyl).

The cation $Y^{\oplus}$ of a pharmaceutically acceptable base is, for example, an alkali metal ion, for example the lithium, sodium or the potassium ion, the ammonium ion, a mono-, di- or tri-$C_1$-$C_4$-alkylammonium ion, for example the trimethyl-, ethyl-, diethyl-, or triethylammoniumion, a 2-hydroxyethyl-tri-$C_1$-$C_4$-alkylammoniumion, for example the choline cation, or is the 2-hydroxyethylammonium-ion, or the cation of a basic aminoacid, for example lysine or arginine.

Preferred are phospholipids of the formula I from natural sources wherein $R_1$ and $R_2$ are different or identical $C_{10}$-$C_{20}$-alkanoyl or $C_{10}$-$C_{20}$-alkenoyl groups, for example n-dodecanoyl, n-tetradecanoyl, n-hexadecanoyl, or n-octadecanoyl, or 9-cis-dodecenoyl, 9-cis-tetradecenoyl, 9-cis-hexadecenoyl, 6-cis-, 6-trans-, 9-cis-, 9-trans-

or 11-cis-octadecenoyl or 9-cis-icosenoyl, for example phosphatidylserine from bovine brain, and synthetic phospholipids of the formula I wherein $R_1$ and $R_2$ are identical $C_{10}$-$C_{20}$-alkenoyl groups, for example 9-cis-hexadecenoyl, 6-cis-, 6-trans-, 9-cis-, 9-trans- or 11-cis-octadecenoyl, for example sodium-1,2-di-(9-cis-octadecenoyl)-3-sn-phosphatidyl-( S)-serine.

In a phospholipid of the formula II (component c)) the group -($C_nH_{2n}$)- is straight chain or branched alkylene, for example 1,1-, 1,2- or 1,3-propylene or 1,2-, 1,3- or 1,4-butylene or, preferably, 1,2-ethylene (n=2).

In a phospholipid of the formula II the groups $R_3$, $R_4$ and $R_5$ preferably are hydrogen or methyl.

Preferred are phospholipids of the formula II, wherein $R_4$, $R_5$ and $R_6$ are hydrogen or methyl, from natural sources from plants or from animals, and wherein $R_1$ and $R_2$ are different or identical $C_{10}$-$C_{20}$-alkanoyl or $C_{10}$-$C_{20}$-alkenoyl groups, for example n-dodecanoyl, n-tetradecanoyl, n-hexadecanoyl, or n-octacedanoyl, or 9-cis-dodecenoyl, 9-cis-tetradecenoyl, 9-cis-hexadecenoyl, 6-cis-, 6-trans-, 9-cis-, 9-trans- or 11-cis-octadecenoyl, or 9-cis-icosenoyl, for example lecithin or cephalin from chicken eggs or soy bean lecithin, synthetic phospholipids (II) wherein $R_1$ and $R_2$ are identical $C_{10}$-$C_{20}$-alkanoyl groups, and synthetic phospholipids (II), wherein $R_1$ is $C_{10}$-$C_{20}$-alkanoyl, for example n-dodecanoyl, n-tetradecanoyl, n-hexadecanoyl, or n-octadecanoyl, and $R_2$ is $C_{10}$-$C_{20}$-alkenoyl, for example 9-cis-hexadecenoyl, 6-cis-, 6-trans-, 9-cis-, 9-trans- or 11-cis-octadecenoyl, especially 1-n-hexadecanoyl-2-(9-cis-octadecenoyl)-3-sn-phosphatidylcholine.

Aqueous liposome dispersions wherein the phospholipids of the formulae I and II are the encapsulating material and a muramylpeptide of the formula III in combination with hybrid-interferon is encapsulated, optionally after concentration or isolation of the liposomes, for example in the ultracentrifuge, are suitable for therapeutic purposes for oral (p.o.) or parenteral (bukkal, lingual, sublingual, i.v., i.c., epicutane, s.c., i.m. or especially nasal) administration.

For intrabladder administration, the liposome-containing aqueous dispersion can be mixed with pharmaceutically acceptable diluents or carriers or with customary additives.

For parenteral administration (epicutane) the liposome-containing aqueous dispersion can be mixed with customary thickeners, for example hydroxypropylcellulose, suitable preservatives, antioxidants and perfumes, and can be used in the form of a lotion or a gel for application to the skin or mucous membranes.

For parenteral administration, the aqueous dispersion of the enriched liposomes can be suspended in a suitable carrier liquid, for example sterile, calcium free, isotonic sodiumchloride or glucose solution, optionally buffered to pH 7.2-7.4.

The pharmaceutical composition according to the present invention may consist of a "kit of parts" set comprising vials or bottles containing the component a), and, separately, vials or bottles containing a homogeneous mixture, for example a lyophilisate, of the phospholipids (I) and (II).

The present invention preferably relates to the described pharmaceutical administration systems buffered from pH 7.2 to 7.4.

Preferred is a phospholipid of the formula I wherein n is one, $R_1$ and $R_2$ are acyl each having 10 to 20 carbon atoms and $Y^{\oplus}$ is the sodium ion, and a phospholipid of the formula II wherein n is two, $R_1$ and $R_2$ are acyl each having 10 to 20 carbon atoms and $R_3$ to $R_5$ represent hydrogen or methyl, in particular a synthetic phospholipid of the formula I wherein n is one, $R_1$ and $R_2$ are identical $C_{10}$-$C_{20}$-alkenoyl groups, for example 9-cis-hexadecenoyl or 6-cis-, 6-trans-, 9-cis-, 9-trans- or 11-cis-octadecenoyl and $Y^{\oplus}$ is the sodium ion, and a synthetic phospholipid of the formula II, wherein $R_1$ and $R_2$ are identical $C_{10}$-$C_{20}$-alkanoyl groups, for example n-dodecanoyl, n-tetradecanoyl, n-hexadecanoyl, or n-octadecanoyl, and $R_3$ to $R_5$ represent methyl, and most preferably synthetic, essentially pure sodium-1,2-di-(9-cis-octadecenoyl)-3-sn-phosphatidyl-(S)-serine (I), and synthetic, essentially pure 1-n-hexadecanoyl-2-(9-cis-octadecenoyl)-3-sn-phosphatidylcholine (II), wherein the molar ratio of the phospholipid component b) to the phospholipid component c) is about 30 to 70 mole per cent.

The pharmaceutical administration systems suitable for application in the form of liposomes are manufactured by preparing a homogeneous mixture of the phospholipids of the formulae I and II and dispersing the homogeneous mixture obtained in an aqueous phase containing a interferon-$\alpha$ and, if necessary, buffering the aqueous dispersion to pH 7.0 to 7.8 and, optionally, concentrating and/or separating the liposomes obtained.

The homogeneous mixture of the phospholipids is prepared by formation of a film or a lyophilisate of the phospholipids. The film is prepared by dissolving the phospholipids (I) and (II) in an organic solvent and stripping the solvent.

Suitable solvents are, for example, unsubstituted or substituted, for example halogenated, aliphatic or cycloaliphatic hydrocarbons, for example n-hexane, cyclohexane, methylenechloride, or chloroform, alcohols, for example methanol or ethanol, lower alkanecarboxylic acid esters or amides, for example acetic acid ethylester or dimethylformamide, or ethers, for example diethylether, tetrahydrofurane or dioxane, or mixtures of these solvents.

The organic solvent is subsequently stripped by applying a vacuum, preferably a high vacuum, or by blowing off with an inert gas, for example nitrogen. The lyophilisate is formed by lyophilizing in a conventional manner a solution of the phospholipids (I) and (II) in an organic solvent according to the method as described in the U.S. Patent Specification No. 4,311,712. Suitable solvents are in the solid form together with the phospholipids (I) and (II) at the temperature of the lyophilisation process and are having a melting point of more than 0°C, for example glacial acetic acid, benzene or dioxane, especially tertbutanol.

A homogeneous mixture may also be prepared by spray-drying a solution of the phospholipids (I) and (II) in

an organic solvent having a low boiling point such as chloroform. A powder is obtained by this method.

The ratio of the phospholipid component (I) to the phospholipid component (II) in the homogeneous mixture is approximately 10 v. 90 up to 50 v. 50 mole per cent. Preferred is the ratio 30 v. 70 mole per cent. The approximate ratio of the molar amounts of the encapsulated material divided by the total amount of the phospholipids (I) and (II) is about 0.0001 to 0.1 v. 1.0, preferably 0.005 to 0.01 v. 0.1.

The dispersion is carried out by adding the homogeneous mixture of the phospholipids (I) and (II) to the aqueous phase containing the muramylpeptide (III) and interferon-α hybrid and by agitation of the aqueous phase (vigorous shaking - Vortex mixer or stirring at high speed). A mixture of small, large, unilamellar or multilamellar liposomes is formed spontaneously at a high rate without supplying external energy. Approximately 0.1 to 40 per cent per weight, preferably 2 to 20 per cent per weight, of the homogeneous mixture relative to the total weight of the aqueous dispersion can be dispersed in the aqueous phase. Preferably, such dispersions are further diluted to about 1 micromole lipid per ml. The liposome dispersions of that concentration have entrapped approximately 2.5 microliters of the aqueous phase per micromole of the lipid.

The preparation of the pharmaceutical compositions according to the present invention in the form of liposomes can also be carried out by other methods known in the art for preparing liposomes, for example by sonication with supersonic waves, by infusion methods or reversed phase evaporation.

The dispersion step is performed at temperatures below 60°, preferably at room temperature. In view of a potential thermal sensitivity of the encapsulated material, the dispersion is carried out under cooling and, optionally, under inert gas atmosphere, for example nitrogen or argon atmosphere.

The liposomes obtained can be made storage stable in the aqueous phase up to several weeks or months after addition of stabilizers, for example mannite or lactose.

The size of the liposomes formed depends, inter alia, on the structure of the active ingredient and the lipid component, the mixing ratio of the components and the concentration of these components in the aqueous dispersion. Thus, for example, by increasing or reducing the concentration of the lipid component it is possible to produce aqueous phases having a high content of small or large liposomes. Large multilamelar liposomes are preferred.

The separation of small liposomes from large liposomes, if desired, is effected by means of conventional separation methods, for example sedimentation of the large liposomes in an ultracentrifuge, gel filtration or extrusion through straight-pored filters. For example, on centrifuging, for example from 5 to 30 minutes in a rotational field giving rise to an inertial force equivalent to a gravitational field of 5000-40 000 x g, large liposomes are deposited, whilst small liposomes remain dispersed and can be decanted off. After repeated centrifugation, complete separation of the large liposomes from the small liposomes is achieved.

Liposomes in the aqueous phase having a diameter greater than $6.0 \times 10^{-8}$ m, for example large multilamellar liposomes, can be separated off by gel filtration, for example with Sepharose or Sephacryl as carriers, or by use of polycarbonate filters with defined size of the pores, e.g. with a cutoff size of $<5$ μm.

By extrusion through straight-pored filters, for example membrane filters of the Nucleopore® or polycarbonate type having a pore diameter of approximately $1.0 \times 10^{-7}$ - $1.0 \times 10^{-9}$ m at a pressure of approximately from 0.1 to 1.5 bar and a filtration rate of approximately 20 ml/h, it is possible to obtain a particularly uniform size distribution of the liposomes.

The formation of liposomes and their content in the aqueous phase can be detected in a manner known per se by using various physical analytical methods, for example by microscopy of freeze-fracture samples and thin sections in an electron microscope, by X-ray defraction, by dynamic light scattering, by mass determination of the filtrate in an analytical ultracentrifuge and, especially, by spectroscopy, for example in the nuclear magnetic resonance spectrum ($^{1}H$, $^{13}C$ and $^{31}P$).

The phospholipids of the formulae I and II are all known. Some of them are commercially available (Avanti, Fluka, Serva). The preparation of 1,2-di-(9-cis-octadecenoyl)-3-sn-phosphatidyl-(S)serine and of analogous lipids has been described by Browning J. and Seelig J. in Chem. and Phys. of Lipids 24 (1979) 103-118.

The buffer solutions of pH 7,0 to 7,8 preferably are sterile phosphate buffer solutions based on the dihydrogenphosphate/ hydrogenphosphate equilibrium ($KH_2PO_4/Na_2HPO_4$). The preparation of these buffer solutions is described in standard manuals, for example "Hager's Handbuch der Pharmazeutischen Praxis", Springer Verlag, Vol. 1, pg. 357-359. Especially sterile, isotonic calcium-free buffer solution of pH 7.2 (Dulbecco) or Hank's Balanced Salt Solution (M.A. Bioproducts, Walkersville Md. USA) is used.

Accordingly the invention concerns also a pharmaceutical composition, preferably in liposomes form, for the treatment of bladder cancer, comprising a therapeutically effective amount of IFN-α BBDD or BBDB hybrid.

The invention also concerns a method for producing a pharmaceutical composition fo the treatment of bladder cancer, characterized in that a therapeutically effective amount of a IFN-α BBDD or BBDB hybrid is admixed with a pharmaceutically acceptable carrier.

Based on the present experimental results it is estimated that the highest dose to be applied to a human of about 70 kg weight is about 500 mg to 1000 mg liposomes containing $10^7$ to $10^8$ units of an interferon-α hybrid. For invasive and metastatic bladder cancer intravesicle administration in a dosage of 0.5 to 2 mg/per 70 kg of body weight is envisaged. The highest and lowest dose of the encapsulated material, the concentration of the phospholipids in the aqueous phase as well as the proportions of the lipid components (I) and (II) can be varied according to results to be established experimentally in clinical trials.

The particular mode of administrations and the dosage will be selected by the attending physician taking into account the particulars of the patient, the disease and the disease state involved. For instance, treatment of superficial bladder cancer typically involves daily or multidaily doses over weeks or months. The same daily dose levels, viz. about $10^7$ to $10^8$ IU (international units), may be applied.

The invention concerns further the use of the pharmaceutical compositions for the treatment of bladder cancer, especially superficial bladder cancer.

The invention concerns also the IFN-α BBDD or BBDB hybrid for the manufacture of a medicament for the treatment of bladder cancer, especially superficial bladder cancer, which latter may contain instructions for its use, and wherein the cytostatic hybrid may be used in a lower than normally applied dose.

The invention concerns also a preparation or pack comprising IFN-α BBDD or BBDB hybrid and a liposome preparation, for the treatment of bladder cancer, especially superficial bladder cancer and which may include instructions for use.

The invention is based on the following experimental methods and results.

## Experimental Methods and Results

### a) Cell Lines

Human cell lines used were: 253J bladder carcinoma, J-82 (ATCC HTB1), HT-1376 (CRL 1472), RT-4 (ATCC HTB 2), T-24 (ATCC HTB4) and TCCSUP (ATCC HTB5) bladder carcinoma. All cell lines were maintained in mono-layer cultures on plastic in Eagle's minimum essential medium (MEM; Hazelton, Denver, CO) supplemented with 10 % fetal bovine serum (FBS), sodium pyruvate, nonessential amino acids, L-glutamine, and a twofold vitamin solution (GIBCO, Grand Island, NY). The cell lines were confirmed to be free of mycoplasma and pathogenic mouse viruses (Microbiology Associates, Walkersville, MD).

Hybrids of human lymphoblastoid IFN-α $B_1B_2D_3D_4$ and $B_1B_2D_3B_4$ were constructed by recombinant genetic engineering techniques in yeast as described previously (38, 42).

The IFN hybrids were affinity-purified using monoclonal antibody SA-144 (43) exactly as previously described (44). The active IFN fractions were dialyzed against PBS. The protein content of the purified IFN was determined as described by Bradford (45) with bovine serum albumin as a reference. Sodium dodecyl sulfate gel electrophoresis revealed a purity of >90%. All IFN-α hybrids were free of endotoxins as determined by the Limulus amebocyte lysate assay (<0.1 ng/ml) (Cape Cod Associates, Woods Hole, MA).

### b) Iodination of the Interferon Hybrids

Human IFN-α hybrids BBDD and BBDB were iodinated as described by Mogensen and Bandu (46).

The iodinated protein was purified on a Sephadex G-25 column equilibrated with 10 ml PBS and 0.01 % BSA. The initial specific activities of the $^{125}$I-labeled IFN-α $B_1B_2D_3D_4$ and $D_1D_2B_3B_4$ hybrids were 11.5 and 26 μCi/μg protein, respectively. The antiviral activities of the radiolabeled hybrids remained at >90%.

### c) Binding of $^{125}$I-labeled IFN-α Hybrids to Target Cells

Tumor cells were seeded into 24-well plates (16 mm in diameter; Corning, (Corning, NY) at the density of 5 x $10^5$ cells/well. A dense monolayer was obtained subsequent to 6 hours incubation in supplemented medium. At this time, 500 μl of radiolabeled IFN-α $B_1B_2D_3D_4$ or BBDB hybrids were added with or without their respective competitors. Cells were incubated for 60 minutes at 37°C because preliminary experiments revealed that this incubation period produced optimal binding of the BBDD hybrid. Cultures were then washed three times with PBS and lysed with 0.1 N NaOH, and radioactivity was monitored in a gamma counter (LKB, Sweden). All experiments were carried out in triplicate. The results are expressed in percent binding of $^{125}$I-labeled IFN in the presence of increasing concentrations of nonlabeled IFN.

### d) Antiproliferative Activity of Alpha Interferon Hybrids

Tumor cells in exponential growth phase were harvested by a brief treatment with 0.25 % trypsin and seeded into 38-mm$^2$ wells of flat-bottomed 96-well microtiter plates. Our previous experience (10) has shown that optimal starting cell concentrations vary according to the length of the assay. Thus, for a 3-day assay, we seeded 5000 cells per well, and for assays of 5 days' duration, the number was 2500 cells/well. After 18 hours' incubation, the cultures were washed and refed with 100 μl of supplemented MEM. IFN hybrids were added to each well in a volume of 100 μl at the indicated concentrations. After 72 hours and 120 hours, the antiproliferative activity of the IFN hybrids was determined by monitoring the number of metabolically active cells. This was accomplished by the incorporation of the fluorescent vital dye hydroethidine (HET, Polysciences, Worrington, PA), exactly as already described (10, 47). The fluorescent emission of HET was measured by an automatic microfluor reader (MicroFluor®, Dynatech, Alexandria, VA) at a rate of 96 samples per 30 seconds (47). The results were expressed as relative fluorescent units (RFU). RFU of the alpha-IFN-treated cultures were compared with those monitored for control cultures incubated in supplemented medium. The results werde calculated as follows: % cytostasis = 1-[A/B] x 100, where A = RFU (relative fluorescent units) of samples with interferon and B = RFU of medium control.

e) Other Reagants

Eagle's minimum essential medium was purchased from M.A. Bioproducts (Walkersville, MD). Recombinant human IFN-α A was obtained from Hoffman LaRoche (Basel, Switzerland). A stock solution of HET was prepared by dissolving 7 mg of HET in 1 ml of N,N-dimethylacetamide (Polysciences, Inc., Warrington, PA). The working solution (28 µg/ml) was prepared by stirring 40 µl of stock solution into 10 ml of PBS containing $CA^{2+}$ and $Mg^{2+}$. The solution was then filtered through a 0.2 µm Millipore filter (47).

Cytostatic Effects of IFN-α Hybrids on Human Tumor Cell Lines

In the first set of experiments the level of antiproliferative activity mediated by the IFN-α hybrids $B_1B_2D_3D_4$, $B_1B_2D_3B_4$, $D_1D_2B_3B_4$, $B_1D_2B_3B_4$, and IFN-α A were compared. Six different human bladder cancer cell lines were incubated with the IFN hybrids at different concentrations for 72 and 120 hours. The experiment was carried out at least six times. The results were very reproducible and the data are summarized in Tables 1 to 6. The IFN-α hybrids exhibited significant cytostatic effects against all six human cell lines. The effect was most pronounced against all bladder carcinoma cell lines. The bladder carcinoma cell lines were not at all affected by the IFN-α $D_1D_2B_3B_4$ hybrid. In general, the IFN-α $B_1B_2D_3D_4$ exhibited 5-10-fold higher activity than the IFN-α $D_1D_2B_3B_4$ hybrid. IFN-α hybrid $B_1D_2B_3B_3$ and IFN-α A produced marginal cytostatic activity in the 3-day assay. In order to exclude the possibility that an interaction time of 72 hours was not sufficient for full expression of the activity of the IFN-α hybrids, we incubated the cell lines with IFN-α for up to 120 hours. A marked increase in the cytostatic activity of IFN-α $B_1B_2D_3D_4$ hybrid was observed (as compared with 3 days' incubation). In contrast, the activity of IFN-α $D_1D_2B_3B_4$ hybrid was still negligible, suggesting that the low activity of the IFN-α $D_1D_2B_3B_4$ hybrid was not due to insufficient interaction time. IFN-α $B_1D_2B_3B_4$ and IFN-α A exhibited similar cytostatic activities, which were higher than IFN-α $D_1D_2B_3B_4$, but lower than IFN-α $B_1B_2D_3D_4$.

Table 1

Cell Line: J-82 Bladder Cancer

| IFN-α Hybrid | Concentration Units/ml | Percent Cytostasis |
|---|---|---|
| BBDD | 1000 | 47 |
| | 100 | 29 |
| | 10 | 23 |
| | 1 | 3 |
| BBDB | 1000 | 52 |
| | 100 | 30 |
| | 10 | 28 |
| | 1 | 2 |
| BDBB | 1000 | 27 |
| | 100 | 20 |
| | 10 | 14 |
| | 1 | 8 |
| Roche-A | 1000 | 13 |
| | 100 | 9 |
| | 10 | 9 |
| | 1 | 2 |
| DDBB | 1000 | 9 |
| | 100 | 8 |
| | 10 | 2 |
| | 1 | 2 |

3 day assay for growth inhibition.

Percent cytostasis as compared to control cultures (in medium).

Table 2

Cell Line: HT-1376 Bladder Cancer

| IFN-α Hybrid | Concentration Units/ml | Percent Cytostasis |
|---|---|---|
| BBDD | 1000 | 22 |
| | 100 | 12 |
| | 10 | 17 |
| | 1 | 11 |
| BDBB | 1000 | 13 |
| | 100 | 12 |
| | 10 | 10 |
| | 1 | 0 |
| Roche-A | 1000 | 13 |
| | 100 | 12 |
| | 10 | 6 |
| | 1 | 4 |
| DDBB | 1000 | 14 |
| | 100 | 10 |
| | 10 | 8 |
| | 1 | 2 |

3 day assay for growth inhibition.

Percent cytostasis as compared to control cultures (in medium).

Slow growing cell line.

Table 3

Cell Line: RT-4 Bladder Cancer

| IFN-α Hybrid | Concentration Units/ml | Percent Cytostasis |
|---|---|---|
| BBDD | 1000 | 98 |
| | 100 | 28 |
| | 10 | 24 |
| | 1 | 9 |
| BDBB | 1000 | 22 |
| | 100 | 13 |
| | 10 | 15 |
| | 1 | 2 |
| Roche-A | 1000 | 23 |
| | 100 | 12 |
| | 10 | 4 |
| | 1 | 0 |
| DDBB | 1000 | 8 |
| | 100 | 6 |
| | 10 | 9 |
| | 1 | 2 |

3 day assay for growth inhibition.

Percent cytostasis as compared to control cultures (in medium).

Sensitive cell line.

Table 4

Cell Line: 253J Bladder Cancer

| IFN-α Hybrid | Concentration Units/ml | Percent Cytostasis |
|---|---|---|
| BBDD | 1000 | 64 |
| | 100 | 36 |
| | 10 | 27 |
| | 1 | 9 |
| BBDB | 1000 | 82 |
| | 100 | 60 |
| | 10 | 48 |
| | 1 | 16 |
| BDBB | 1000 | 46 |
| | 100 | 43 |
| | 10 | 17 |
| | 1 | 7 |
| Roche-A | 1000 | 39 |
| | 100 | 32 |
| | 10 | 20 |
| | 1 | 10 |
| DDBB | 1000 | 11 |
| | 100 | 10 |
| | 10 | 10 |
| | 1 | 8 |

3 day assay for growth inhibition.

Percent cytostasis as compared to control cultures (in medium).

Table 5

Cell Line: T-24 Bladder Cancer

| IFN-α Hybrid | Concentration Units/ml | Percent Cytostasis |
|---|---|---|
| BBDD | 1000 | 44 |
| | 100 | 27 |
| | 10 | 26 |
| | 1 | 15 |
| BDBB | 1000 | 21 |
| | 100 | 20 |
| | 10 | 17 |
| | 1 | 6 |
| Roche-A | 1000 | 33 |
| | 100 | 21 |
| | 10 | 14 |
| | 1 | 6 |
| DDBB | 1000 | 16 |
| | 100 | 13 |
| | 10 | 10 |
| | 1 | 0 |

3 day assay for growth inhibition.

Percent cytostasis as compared to control cultures (in medium).

Table 6

Cell Line: TCCSUP Bladder Cancer

| IFN-α Hybrid | Concentration Units/ml | Percent Cytostasis |
|---|---|---|
| BBDD | 1000 | 56 |
| | 100 | 50 |
| | 10 | 31 |
| | 1 | 1 |
| BDBB | 1000 | 30 |
| | 100 | 28 |
| | 10 | 16 |
| | 1 | 9 |
| Roche-A | 1000 | 32 |
| | 100 | 12 |
| | 10 | 4 |
| | 1 | 4 |
| DDBB | 1000 | 18 |
| | 100 | 14 |
| | 10 | 1 |
| | 1 | 6 |

3 day assay for growth inhibition.

Percent cytostasis as compared to control cultures (in medium).

In another set of experiments extenting over four days the results compiled in Table 7 have been obtained.

Table 7: 3 different human bladder cancer cell lines

| IFN-α Hybrid | Concentration Units/ml | Percent Cytostasis against | | |
|---|---|---|---|---|
| | | 253J | T-24 | TCC |
| BBDD | 10 | 18 | 6 | – |
| | 100 | 45 | 34 | – |
| | 500 | 50 | 30 | – |
| BBDB | 10 | 33 | 30 | 4 |
| | 100 | 62 | 46 | 40 |
| | 500 | 60 | 42 | 40 |
| BDBB | 10 | 6 | 18 | 0 |
| | 100 | 22 | 42 | 10 |
| | 500 | 44 | 26 | 25 |
| DDBB | 10 | 8 | 30 | 5 |
| | 100 | 15 | 30 | 30 |
| | 500 | 17 | 20 | 30 |

4 days assay.

Percent cytostasis as compared to control cultures (in medium).

Antiproliferative Effects of IFN-α Hybrid BBDB in Liposomes on Human Tumor Cell Lines

In a further set of experiments the antiproliferative effects of IFN-α BBDB encapsulated in liposomes on the human bladder cancer cell line 253J and the IFN-α BBDB resistant cell line 253J-IFN-resistant (253J-IFN-$\alpha^R$) were measured and compared with the antiproliferative effects of IFN-α BBDB alone, a mixture of liposomes and not-encapsulated IFN-α BBDB, liposomes alone and also with the anticancer drug adriamycine as the positive control.

The IFN-α hybrid was encapsulated in the multilamelar liposomes PC/PS (7:3) as described e.g. in Example 1. IFN-α BBDB associates with PC/PS liposomes in a hydrophilic manner to a calculated aqueous volume of 2-2.5 ml/µmol lipid. For intravesicular administration, it is important that the liposomes with the IFN-α be relatively stable in human urine. Corresponding tests with 125-I labeled IFN-α BBDB in serum-free medium or medium containing 5 % fetal calfserum, indicated that for four hours, liposomes containing radioactive IFN-α BBDB were stable and did not release the molecule.

The IFN-α resistant cell line (253J-IFN-$\alpha^R$) was selected from the cell line 253J (available from ATCC) growing in 10.000 U IFN-α BBDB/ml for several weeks. Accordingly, in contrast to the IFN-α sensitive cell line 253J, the resistant cell line can grow in the presence of 10.000 U/ml of IFN-α BBDB.

Percent cytostasis was determined and calculated according to the 96-120 hour HET assay described herein before. The results compiled in Figures 1 to 4 are unexpected and surprising.

Short description of the Figures

Figure 1: Displayed are the antiproliferative effects, of IFN-α BBDB free (I) and encapsulated in liposomes (L/I), liposomes (with Hank's balanced salt solution, HBSS) plus free IFN-α BBDB (L+I), liposomes in HBSS without IFN-α (L) and adriamycin (ADR) against the human bladder tumor cell line 253J expressed in percent cytostasis at IFN-α concentrations of 1000, 100 and 10 U/ml. Three out of nine experiments of the 96-120 hour HET assay are shown.

Figure 2: Displayed are the antiproliferative effects in the 96-120 hour HET assay of free IFN-α BBDB (I), liposomes with encapsulated IFN-α Hybrid (L/I), liposomes with HBSS plus free IFN-α BBDB (L+I), liposomes in HBSS without IFN-α (L) and adriamycin (ADR) against the human bladder tumor cell line $253J^R$ expressed in percent cytostasis at IFN-α concentrations of 1000, 100 and 10 U/ml. Three experiments are shown.

Figure 3: Displayed are the antiproliferative effects against the human bladder tumor cell line 253J, expressed in percent cytostasis, in the 96-120 hour HET assay of IFN-α BBDB at high (22.000 U/ml, I-Hi) and low (1000 U/ml, I-LO) doses, liposomes-HBSS plus 22.000 U/ml of free IFN-α BBDB (L+I-Hi), liposomes-HBSS plus 1000 U/ml of free IFN-α BBDB (L+I-Lo), 4400 U/ml of IFN-α-BBDB encapsulated in liposomes (L/I-Hi), 200 U/ml of IFN-α BBDB encapsulated in liposomes (L/I-Lo) and liposome-HBSS without IFN-α BBDB (L). The results of three experiments each at 2 and 4 hour exposure time are shown.

Figure 4: Displayed are the antiproliferative effects against the IFN-α resistant human bladder tumor cell line $253J^R$, expressed in percent cytostasis, in the 96-120 hour HET assay of IFN-α BBDB at high (22.000 U/ml, I-Hi) and low (100 U/ml, I-Lo) doses, liposomes-HBSS plus 22.000 U/ml of free IFN-αBBDB (L+I-Hi), liposomes-HBSS plus 1000 U/ml of free IFN-α BBDB (L+I-Lo), 4400 U/ml of IFN-α BBDB encapsulated in liposomes (L/I-Hi), 200 U/ml of IFN-α BBDB encapsulated in liposomes (L/I-Lo) and liposome-HBSS without IFN-α BBDB (L). The results of three experiments each at 2 and 4 hour exposure time are shown.

The experiments according to Figure 1 were done nine times with very similar results. Three experiments are shown here. The cell line is sensitive to free IFN-α BBDB (I). At any of the concentrations when IFN-α is presented in liposomes (L/I), there is an increased level of cytostasis. Liposomes with saline (Hank's balanced salt solution, HBSS) and free IFN (L+I) do not produce cytostasis greater than free IFN-α BBDB. This means that liposomes per se are not antagonistic to IFN-α BBDB. Liposomes only (L) are not cytostatic. The positive control is the anticancer drug adriamycin.

The augmented cytostatic activity of liposomal IFN-α BBDB against human bladder tumor appears to correlate with binding of liposomes to cells (as determined by both electron microscopic studies and the binding of radiolabeled lipid).

The study according to Figure 2 used the 253J IFN-$\alpha^R$ cells in three experiments with very similar results. The cells are resistant to free IFN-α BBDB even at 1000 U/ml. When the IFN-α BBDB is presented in liposomes, highly significant cytostasis is found (L/I). The ability of IFN-α BBDB to overcome resistance to IFN-α BBDB is unexpected and very important. The augmented cytostatic activity of liposomal INF-α against human bladder tumor appears to correlate with lipid binding (as determined by both EM examination of the cells and the binding of labeled lipid).

In the studies according to Figures 4 and 5 (3 exprreriments/each), the IFN-α-sensitive 253J and IFN-α-resistant 253J IFN-$\alpha^R$ cells were exposed to free IFN-α BBDB (I) or IFN-α BBDB in liposomes (L/I) at various doses for 2 or 4 hours, and then the cells were washed. Cytostasis was determined 4-5 days later. The results show that even a brief exposure (2 hours) of cells to liposomes with high dose IFN-α BBDB (22.000 U/ml-equivalent) produce highly significant cytostasis even in the IFN-α-resistant cell line.

The cytostatic activity of liposomal-INF-α BBDB is both dose- and time-dependent, and results in up to 2-5 fold greater direct antiproliferative activity than that observed using free IFN-α BBDB in solution. As little as 30 minutes to 4 hours exposure (such as for intravesical therapy of bladder cancer) results in significant cytostasis.

The cytostatic properties of liposomal-IFN-α BBDB are able to overcome the IFN-α resistance observed for a 253J subline selected for the ability to grow in 10.000 U/ml of free IFN-α BBDB.

These studies indicate that

1. IFN-α BBDB in liposomes has 2-5 fold antitumor effects than free IFN-α at equivalent concentration.
2. IFN-α BBDB in liposomes can overcome resistance of tumor cells to free IFN-α.
3. IFN-α BBDB in liposomes can produce antitumor effects even after a 2-hour interaction with bladder cancer cells.
4. IFN-α BBDB in liposomes is stable for at least 4 hours in human urine.

Accordingly the use of liposomes containing IFN-α BBDB is indicated for intravesicular (intrabladder) therapy for superficial human bladder cancer.

The following examples are illustrating the invention further without limiting the scope thereof. Temperatures are given in degrees Celsius.

Further Abbreviations used in the Examples:
MLV: multilaminar vessicle
PC : Phosphatidylcholine
PS : Phosphatidylserine

Example 1:

a) In a round flask 75 mg (95 % purity) sodium-1,2-di-(9-cis-octadecenoyl)-3-sn-phosphatidyl-(S)-serine (PS, prepared according to Browning J. and Seelig J., Chem. and Physics of Lipids 24, 103-118 (1979)) and 175 mg (95 % purity) 1-n-hexadecanoyl-2-(9-cis-octadecenoyl)-3-sn-phosphatidylcholine (PC, Avanti) are dissolved in 586 mg sterile tert-butanol. The solution is filtered under sterile conditions over Acrodisc® (2.0 x $10^{-7}$ m) and is bottled at -45° in a sterile vial. A vacuum of 6.0 x $10^{-5}$ bar is applied to the vial until room temperature is reached to remove the solvent.

b) To this vial containing the lyophilisate of the phospholipids 10 ml sterile calcium-free, phosphate buffered (pH 7.2-7.4) sodium chloride solution (Dulbecco) containing $10^7$ to $10^8$ units of recombined human interferon-α BBDD or BBDB is added with a sterile syringe. The vial is then shaken on a standardized laboratory mixer (Vortex, stage 6). The dispersion containing liposomes is storage stable at 4° and is suitable for intrabladder or parenteral administration (i.v.).

Example 2:

MLV are prepared from a mixture of chromatographically pure distearoyl-PC and PS in a molar ratio of 7:3. IFN-α BBDD is encapsulated within the MLV in a conventional manner according to Example 1 by mechanical agitation on a Vortex mixer. Unencapsulated materials are removed by washing the MLV by centrifugation. The internal aqueous volume of the MLV is determined to be 2.5 ± 0.4 µl/µmol of the phospholipid. The liposome preparations is adjusted to 1 µmole total lipid/ml in media. A liposome concentration of 100 nmoles phospholipid per $10^5$ cells contains approximately 0.25 µl of encapsulated materials.

REFERENCES

1. Goeddel DV, Leung DW, Dull TJ, et al: The structure of eight distinct cloned human leukocyte interferon cDNAs. Nature 290:20-23, 1981
2. Henco K, Brosius A, Fujisawa A, et al: Structural relationship of human interferon alpha genes and pseudogenes. J.Mol.Biol. 185:227-260, 1985
3. Valenzuela D, Weber H, Weissmann C: Is sequence conservation in interferons due to selection for functional proteins? Nature 313:698-700, 1985
4. Isaacs A, Lindenmann J: Virus interference: The interferon. Proc.R.Soc. Lond [Biol] 147:258-267, 1957
5. Horoszewicz JS, Leong SS, Carter WA: Noncycling tumor cells are sensitive targets for the antiproliferative activity of human interferon. Science 290:1091-1094, 1979
6. Denz H, Lechleitner M, Marth C, et al: Effect of human recombinant alpha-2- and gamma-interferon on the growth of human cell lines from solid tumors and hematologic malignancies. J. Interferon Res. 5:147-157, 1985
7. Gresser I: How does interferon inhibit tumor growth? in Gresser I (Ed. in chief) Interferon 6, London, Academic Press, 1985, pp. 1-12
8. Brunda MJ, Wright RB: Differential antiproliferative effects of recombinant interferons alpha and gamma on two murine cell lines. Int. J. Cancer 37:287-291, 1986
9. Rosenblum MG, Maxwell BL, Talpaz M, et al: In vivo sensitivity and resistance of chronic myelogenous leukemia cells to alpha-interferon: Correlation with receptor binding and induction of 2′,5′-oligoadenylate synthetase. Cancer Res. 46:4848-4852, 1986
10. Fidler IJ, Heicappell R, Saiki I, et al: Direct antiproliferative effects of recombinant human interferon-alpha B/D hybrids on human tumor cell lines. Cancer Res. 47, 2020-2077 (1987)
11. De Maeyer-Guignard J, De Maeyer E: Immunomodulation by interferons: Recent developments, in Gresser I (Ed. in chief) Interferon 6, London, Academic Press, 1985, pp. 93-126
12. Ortaldo JR, Herberman RB, Harvey C, et al: A species of human alpha interferon that lacks the ability to boost human killer cell activity. Proc. Natl. Acad. Sci. USA 81:4926-4929, 1984
13. Masucci MG, Szigetti R, Klein E, et al: Effect of interferon alpha 1 from E. coli on some cell functions. Science 209:1431-1435, 1980
14. Talmadge JE, Herberman RB, Chirigos MA, et al: Hyporesponsiveness to augmentation of murine natural killer cell activity in different anatomical compartments by multiple injections of various immunomodulators including recombinant interferons and interleukin 2. J. Immunol. 135:2483-2488, 1985
15. Minato N, Reid L, Cantor H, et al: Mode of regulation of normal killer cell activity by interferon. J. Exp. Med. 152:124-137, 1980
16. Jett JR, Mantovani A, Herberman RB, et al: Augmentation of human monocyte-medited cytolysis by interferon. Cell Immunol. 54:425-434, 1980
17. Candler RV, Rouse BT, Moore Rn: Regulation of interleukin I production by alpha and beta

interferons: Evidence for both direct and indirect effects. J. Interferon Res. 5:179-189, 1985

18. Sone S, Utsugi T, Shirahama T, et al: Induction by interferon of tumoricidal activity of adherent mononuclear cells from human blood: Monocytes as responses and effector cells. J. Biol. Mod. 4:134-140, 1985

19. Schwartz R, Momburg F, Moldenhauer G, et al: Induction of HLA class II antigen expression on human carcinoma cell lines by IFN gamma. Int. J. Cancer 35:245-251, 1985

20. Rossi GB: Interferons and cell differentiation, in Gresser I (ed. in chief), Interferon 6, London, Academic Press, 1985, pp. 31-68

21. Aguet M, Gresser I, Hovanessian AG, et al: Specific-affinity binding of $^{125}$I-labeled mouse interferon to interferon resistant embryonal carcinoma cells in vitro. Virology 114:585-588, 1981

22. Aguet M: High affinity binding of $^{125}$I-labeled mouse interferon to a specific cell surface receptor. Nature 284:453-461, 1981

23. Gordon I, Stevenson D, Tumas V, et al: Mouse interferon receptors: A difference in their response to alpha and beta interferons. J. Gen. Virol. 64:2777-2780, 1983

24. Branca AA, D'Alessandro SB, Baglioni C: Internalization and degradation of human alpha-A interferon bound to bovine MDBK cells: Regulation of the decay and resynthesis of receptors. J. Interferon Res. 3(4):465-471, 1983

25. Yonehara S, Yonehara-Takahashi M, Ishii A, et al: Different binding of human interferon alpha 1 and alpha 2 to common receptors on human and bovine cells. J. Biol. Chem. 258:9046-9049, 1983

26. Merlin G, Falcoff E, Aguet M: $^{125}$I-labeled human interferons alpha, beta and gamma: Comparative receptor-binding data. J. Gen. Virol. 66:1149-1152, 1985

27. Traub A, Feinstein S, Gez M, et al: Purification and properties of the alpha interferon receptor of human lymphoblastoid (Namalva) cells. J. Biol. Chem. 259(22):13872-13877, 1984

28. Faltynek CR, McCandless S, Baglioni C: Single high affinity binding of Interferon alpha 2 to receptor on human lymphoblastoid cells: Internalization and inactivation of receptors. J. Cell Physiol. 123:459-466, 1985

29. Faltynek CR, Princler GL, Rossio JL, et al: Relationship of the clinical response and binding of recombinant interferon alpha in patients with lymphoproliferative diseases. Blood 67(4):1077-1082, 1986

30. Hannigan GE, Gewert DR, Fish EN, et al: Differential binding of human interferon-alpha subtypes to receptors on lymphoblastoid cells. Biochem. Biophys. Res. Commun. 110:537-544, 1983

31. Branca AA, Baglioni C. Evidence that types I and II interferons have different receptors. Nature 294:768-770, 1981

32. Faltynek CR, Princler GL, Ortaldo JR, et al: Expression of IFN-alpha and IFN-gamma receptors on normal human small resting T-lymphocytes and large granular lymphocytes. J. Immunol. 136(11):4134-4139, 1986

33. Razziudin A, Sarkar FH, Dutkowski R, et al: Receptors for human alpha and beta interferon, but not for gamma interferon are specified for by human chromosome 21. Proc. Natl. Acad. Sci. 81:5504-5508, 1984

34. Zoon K, Arnheiter H, Zur-Nedden D, et al: Human interferon alpha enters cells by receptor mediated endocytosis. Virology 130:195-203, 1983

35. Kerr IM, Brown RE: pppA2′p5′A2′p5′A: An inhibitor of protein synthesis synthesized with an enzyme fraction from interferon treated cells. Proc. Natl. Acad. Sci. USA 75:256-260, 1978

36. Zilberstein A, Kimchi A, Schmidt A, et al: Isolation of two interferon-induced translational inhibitors: A protein kinase and an oligoisoadenylate cyclase. Proc. Natl. Acad. Sci. USA 4734-4738, 1978

37. Schmidt A, Chernajovsky Y, Shulman L, et al: An interferon induced phosphodiesterase degrading (2′-5′) oligoadenylate and the C-A terminus of tRNA. Proc. Natl. Acad. Sci. USA 76:4788-4792, 1979

38. Meister A, Uze G, Mogensen KE, et al: Biologic activities and receptor binding of two recombinant interferons and their hybrids. J. Gen. Virol. 67:1633-1643, 1986

39. Koslowski JM, Fidler IJ, Campbell DE, et al: Metastatic behavior of human tumor cell lines grown in the nude mouse. Cancer Res. 44:3522-3529, 1984

40. Naito S, von Eschenbach A, Giavazzi R, et al: Growth and metastasis of tumor cells isolated from a human renal cell carcinoma implanted into different organs of nude mice. Cancer Res. 46:4109-4115, 1986

41. Fidler IJ: Selection of successive tumor lines for metastasis. Nature (New Biology) 242:148-152, 1973

42. Hinnen A, Hicks TB, Fink GR, et al: Transformation in yeast. Proc. Natl. Acad. Sci. USA 75:1929-1933, 1978

43. Alkan SS, Weideli HJ, Schurch AR, et al: Monoclonal antibodies against human leukocyte interferons for a definition of subclasses and their affinity purification, in H Peeters (ed): Protides of Biological Fluids vol. 30. New York, Pergamon Press, pp. 495-498, 1983

44. Staehelin T, Hobs DS, Kung HF, et al: Purification and characterization of recombinant human leukocyte interferon (IFLrA) with monoclonal antibodies. J. Biol. Chem. 256:9750-9754, 1981

45. Bradford, MM: A rapid and sensitive method for the quantitation of microgram quantities of protein utilizing the principle of protein-dye binding. Anal. Biochem. 72:248-254, 1976

46. Mogensen KE, Bandu M-T: Kinetic evidence for an activation step following binding of human interferon alpha 2 to the membrane receptors of Daudi cells. Eur. J. Biochem. 134:355-364, 1983

47. Bucana C, Saiki I, Nayar R, et al: Uptake and accumulation of the vital dye hydroethidine in neoplastic

cells. Histochemistry 34:1109-1115, 1986
48. Borden EC: Interferons and cancer: How the promise is being kept. Interferons J. 43-83, 1984
49. Priestman TJ: Interferons and cancer therapy. J. Pathol. 141:287-295, 1983
50. Gutterman JU, Fein S, Quesada J, et al: Recombinant leukocyte A interferon: Pharmacokinetics, single-dose tolerance, and biologic effects in cancer patients. Ann. Intern. Med. 96:549-556, 1982
51. Merigan TC, Sikora K, Breeden JH, et al: Preliminary observations on the effect of human leukocyte interferon in non-Hodgkin's lymphoma. N. Engl. J. Med. 299:1449-1453, 1978
52. Quesada JR, Swanson DA, Trindale A, et al: Renal cell carcinoma: Antitumor effects of leukocyte interferon. Cancer Res. 43:940-947, 1983
53. Quesada JR, Reuben J, Manning JT, et al: Alpha interferon for induction of remission in hairy-cell leukemia. N. Engl. J. Med. 310:15-18, 1984
54. Friedman RF: Interferon binding: The first step in establishment of antiviral activity. Science 156:1760-1761, 1967
55. Gupta SL, Razziudin A, Sakrar FH: Receptors for human alpha interferon: Are gangliosides involved? J. Interferon Res. 4(3):305-314, 1984
56. Sarkar FH, Gupta SL: Interferon receptor interaction: Internalization of interferon alpha 2 and modulation of its receptor on human cells. Eur. J. Biochem. 140:461-467, 1984
57. Aguet M, Groebke M, Dreiding P: Various human alpha subclases cross react with common receptors: Their binding affinity correlate with their specific biological activity. Virology 132:211-216, 1984

## Claims

1. The use of an IFN-α BBDD or BBDB hybrid for the manufacture of a medicament for the treatment of bladder cancer.
2. The use of an IFN-α BBDD or BBDB hybrid for the manufacture of a medicament for the treatment of superficial bladder cancer according to claim 1.
3. The use of an IFN-α BBDD or BBDB hybrid for the manufacture of a medicament for the treatment of an invasive or metastatic bladder cancer according to claim 1.
4. The use of an IFN-α BBDD or BBDB hybrid for the manufacture of a medicament which is applicable by intrabladder administration, according to claim 1.
5. The use of IFN-α $B_1B_2D_3D_4$ according to claim 1.
6. The use of IFN-α $B_1B_2D_3B_4$ according to claim 1.
7. The use of an IFN-α BBDD or BBDB hybrid for the manufacture of a medicament allowing for administration of the IFN-α hybrid in dosages of $10^7$ to $10^8$ units according to claim 1.
8. A pharmaceutical composition comprising a therapeutically effective amount of an IFN-α BBDD or BBDB hybrid, suitable for the treatment of bladder cancer, which is in the form of a liposome composition.
9. A pharmaceutical liposome composition according to claim 8, consisting of

a) a IFN-α BBDD or IFN-α BBDB hybrid and

b) a phospholipid of the formula

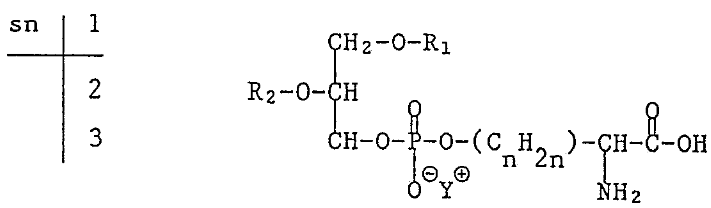

(I),

wherein n is one, two or three, $R_1$ and $R_2$ independently of each other represent alkyl, alkenyl or acyl each having 10-20 carbon atoms, and $Y^{\oplus}$ is the cation of a pharmaceutically acceptable base,

c) a phospholipid of the formula

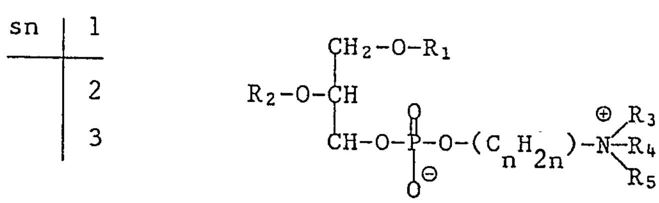

(II),

wherein n is two, three or four, $R_1$ and $R_2$ are defined as above and $R_3$, $R_4$ and $R_5$ represent hydrogen or $C_1$-$C_4$-alkyl, and, optionally, a pharmaceutically acceptable carrier solution buffered to pH 7.0-7.8 and, optionally, pharmaceutically acceptable carriers in solid form.

10. Process for the preparation of a pharmaceutical composition according to any one of claims 8 to 9, characterized in that an IFN-α BBDD or BBDB hybrid are pharmaceutically processed according to conventional methods.

Figure 1

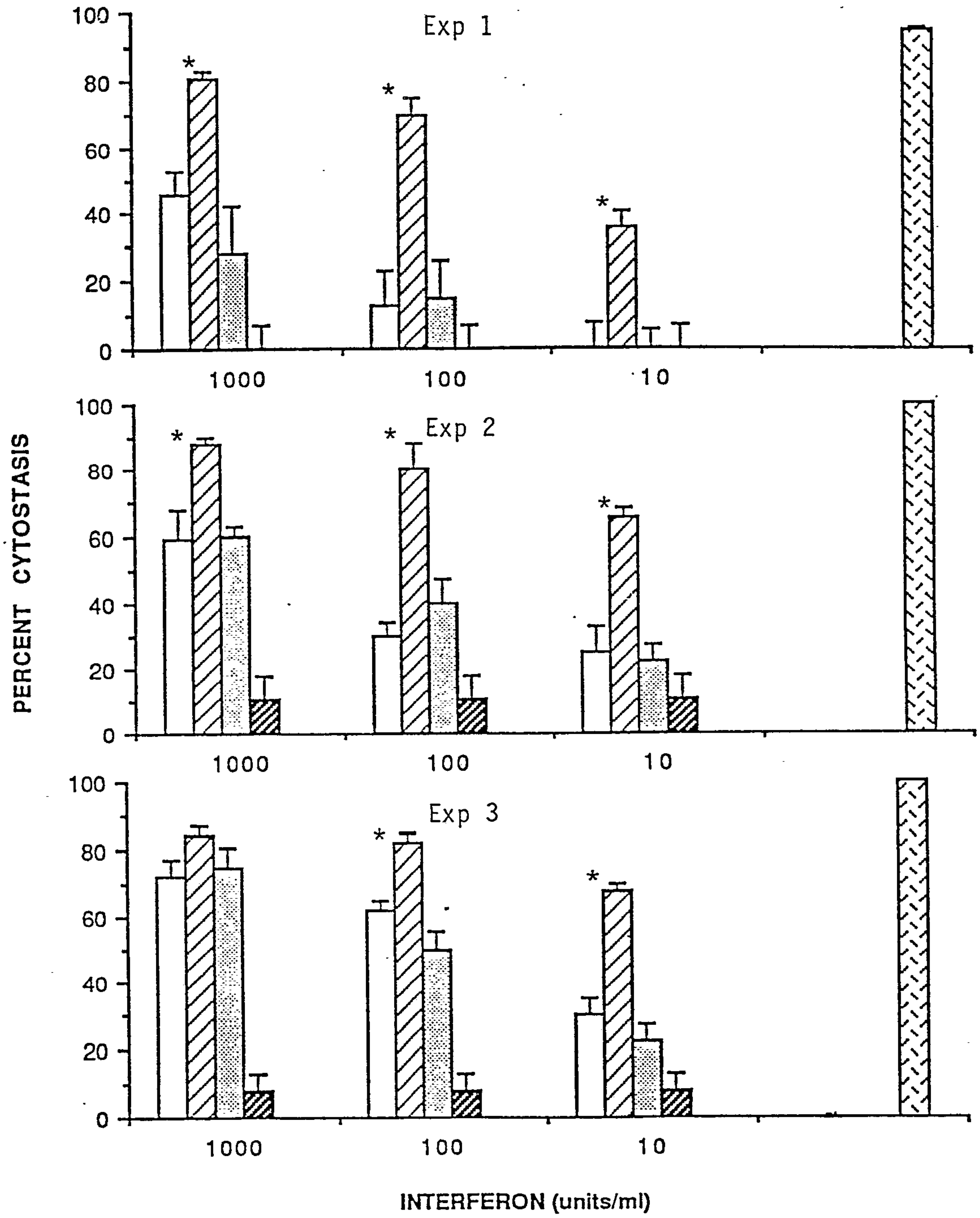


- I = Free IFN-α BBDB
- L/I = Liposome-encapsulated IFN-α BBDB
- L+I = Liposome-HBSS plus free IFN-α BBDB
- L = Liposome-HBSS only, 50 nmol/well
- ADR = Adriamycin, 10 μg/ml

Figure 2

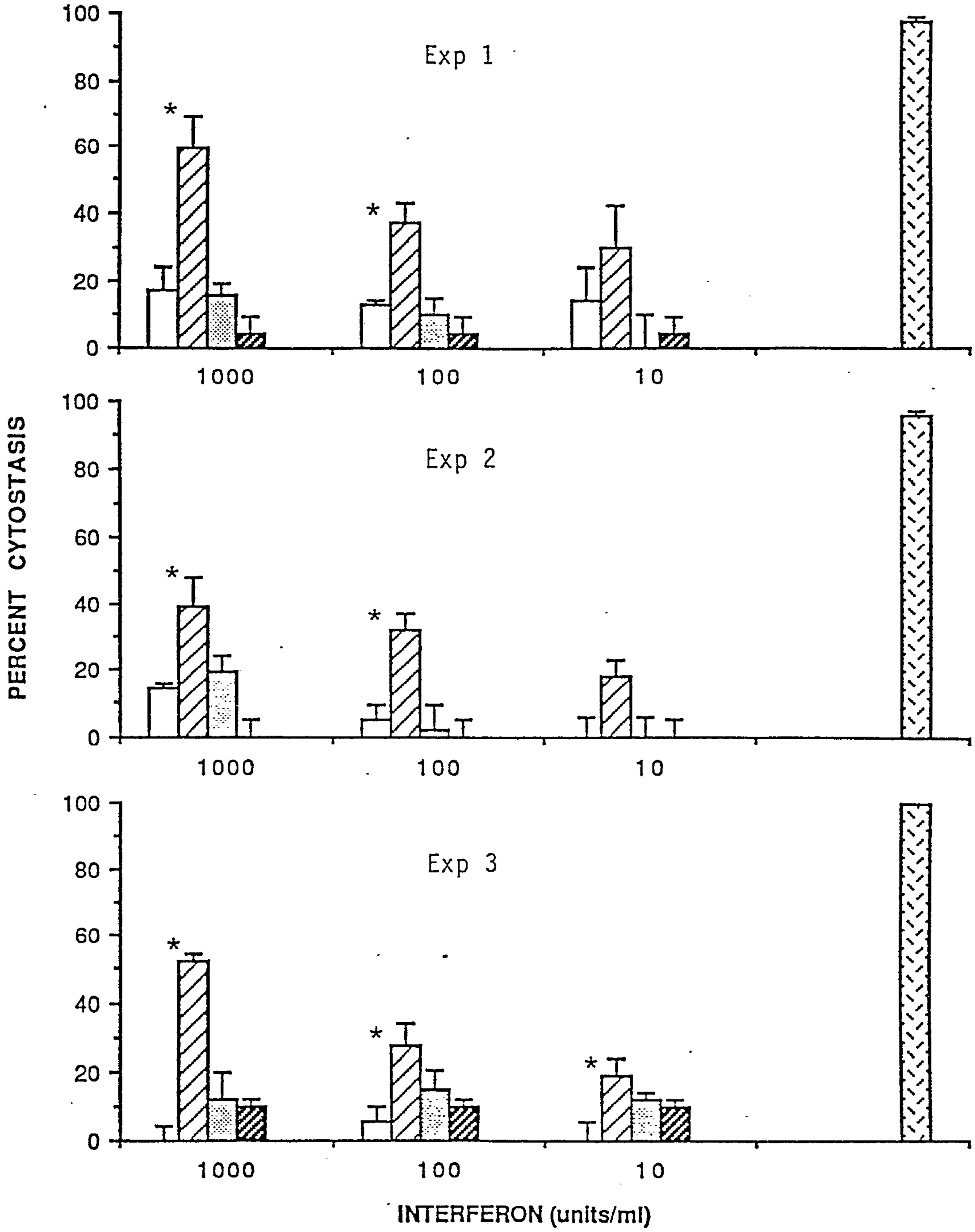


I = Free IFN-α BBDB
L/I = Liposome-encapsulated IFN-α BBDB
L+I = Liposome-HBSS plus free IFN-α BBDB
L = Liposome-HBSS only, 50 nmol/well
ADR = Adriamycin, 10 μg/ml

Figure 3

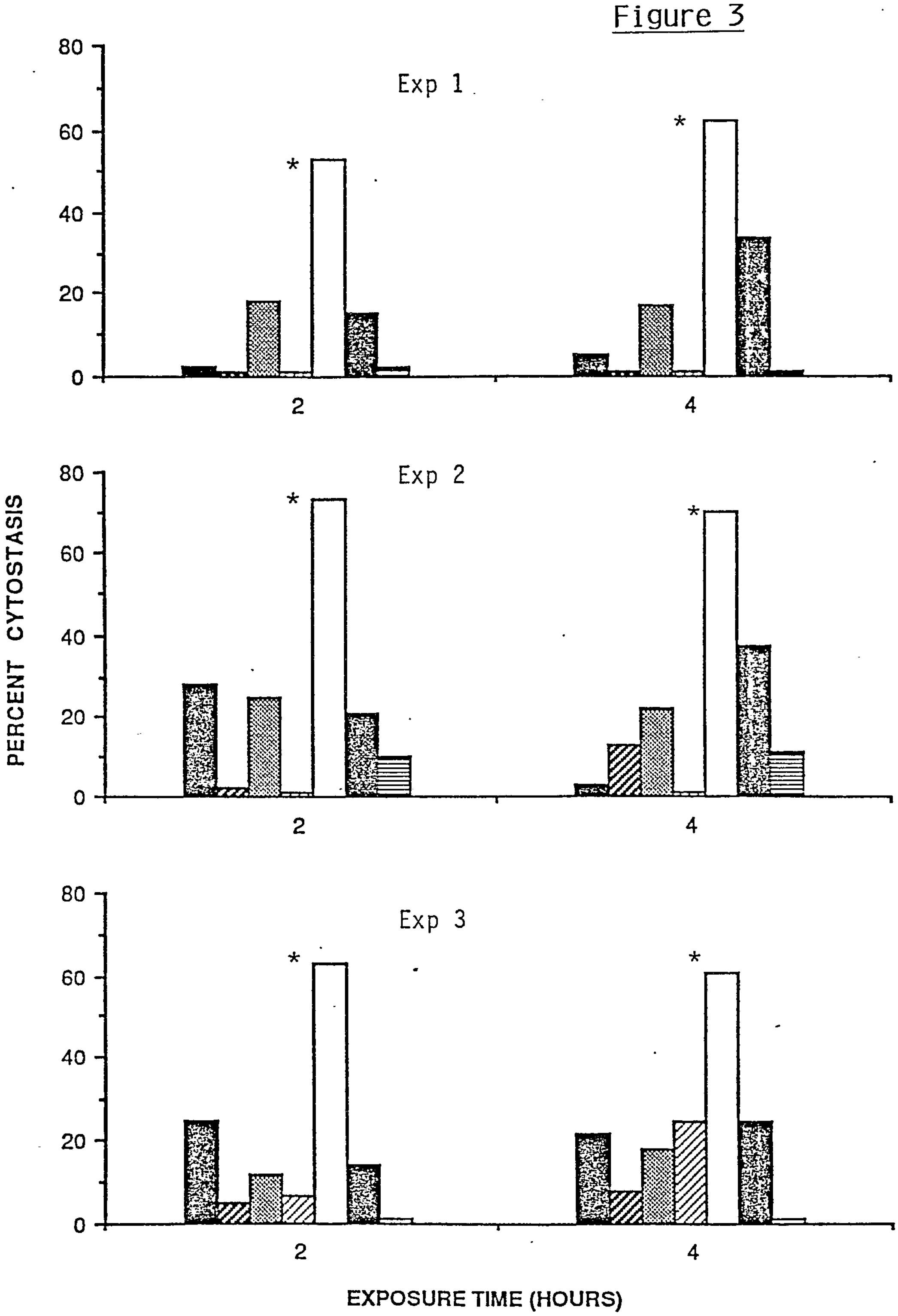


I-HI = Free IFN-α BBDB, 22,000 U/ml
I-Lo = Free IFN-α BBDB, 1,000 U/ml
L+I-Hi = Liposome-HBSS plus free IFN-α BBDB at 22,000 U/ml
L+I-Lo = Liposome-HBSS plus free IFN-α BBDB at 1,000 U/ml
L/I-Hi = Liposome-encapsulated IFN-α BBDB, 4,400 U
L/I-Lo = Liposome-encapsulated IFN-α BBDB, 200 U
L = Liposome-HBSS only, 50 nmol/well

Figure 4

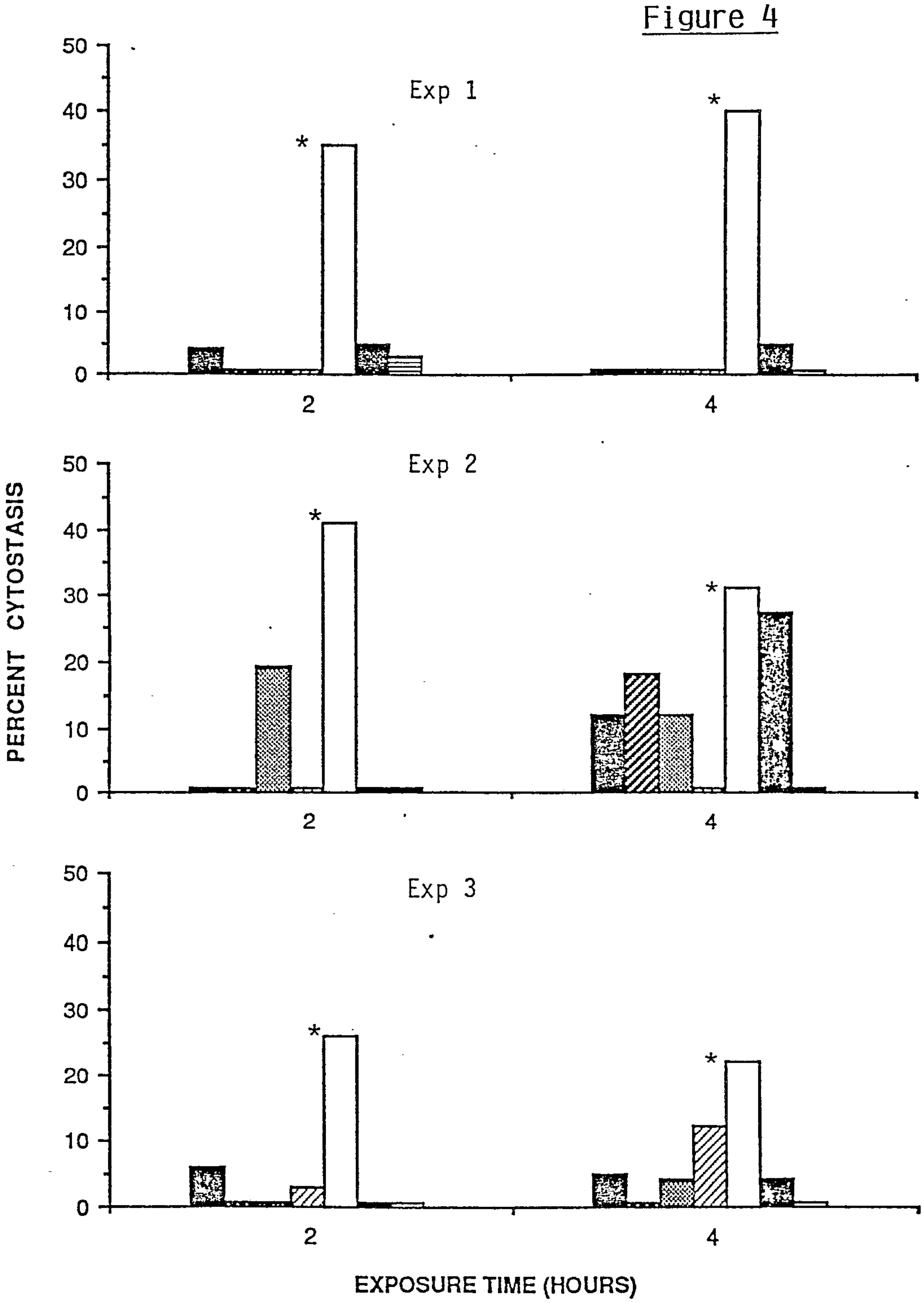


I-HI = Free IFN-α BBDB, 22,000 U/ml
I-Lo = Free IFN-α BBDB, 1,000 U/ml
L+I-Hi = Liposome-HBSS plus free IFN-α BBDB at 22,000 U/ml
L+I-Lo = Liposome-HBSS plus free IFN-α BBDB at 1,000 U/ml
L/I-Hi = Liposome-encapsulated IFN-α BBDB, 4,400 U
L/I-Lo = Liposome-encapsulated IFN-α BBDB, 200 U
L = Liposome-HBSS only, 50 nmol/well